# EUROPÄISCHE PATENTANMELDUNG

(11) **EP 3 604 839 A1**
(43) Veröffentlichungstag der Anmeldung: **05.02.2020**
(21) Anmeldenummer: 18187396.9
(22) Anmeldetag: 03.08.2018
(51) Int. Cl.: F16C 17/02, F16C 41/00, F16D 49/10, F16D 49/20, F16D 11/10, F16D 27/09, F16D 11/00

(54) **LAGERANORDNUNG**

(71) Anmelder: Ondal Medical Systems GmbH, 36088 Hünfeld (DE)
(72) Erfinder: Stroop, Nicolas, 36037 Fulda (DE)
(74) Vertreter: Graf von Stosch Patentanwaltsgesellschaft mbH

(57) **Zusammenfassung**

Lageranordnung (10) mit wenigstens einem ersten Lagerelement (12) und einem zweiten Lagerelement, welche entlang einer gemeinsamen Längsachse (14) relativ rotierbar zueinander miteinander verbunden sind, wobei die Lageranordnung (10) eine Bremsvorrichtung (18) umfasst, welche die relative Rotation der beiden Lagerelemente (12) zueinander mittels einer durch die Bremsvorrichtung (18) erzeugten Reibkraft hemmt, wobei die Bremsvorrichtung (18) wenigstens ein Reibelement (20) und wenigstens eine Spannvorrichtung (22) aufweist, mittels welchem das Reibelement (20) dauerhaft gegen eines der beiden Lagerelemente (12) verspannt ist, und einer Kupplungsvorrichtung (30), welche zwischen einem offenen Zustand und einem geschlossenen Zustand verstellbar ist, wobei in dem geschlossenen Zustand die Bremsvorrichtung (18) eingekuppelt ist, wodurch bei einer Rotation der beiden Lagerelemente (12) relativ zueinander diese Rotation durch die mittels der Bremsvorrichtung (18) erzeugten Reibkraft gehemmt ist, und wobei in dem offenen Zustand die Bremsvorrichtung (18) ausgekuppelt ist.

## Beschreibung

Die Erfindung betrifft eine Lageranordnung, insbesondere mit einer Bremsvorrichtung.

Bei bekannten elektromagnetisch betätigten Lagereinheiten wird eine Ankerplatte mit einem aufgebrachten Reibbelag durch vorgespannte Druckfedern gegen eine Gegenfläche, beispielsweise eine Druckplatte, gepresst, um eine Bewegung jeweiliger Lagerelemente zueinander zu hemmen. Die Ankerplatte ist rotationsfest mit einem ersten Gehäuseteil verbunden, welches als Stator bezeichnet werden kann. Die Druckplatte ist rotationsfest mit einem zweiten Gehäuseteil verbunden, welches nachfolgend als Rotor bezeichnet werden kann. Der Reibbelag kann dabei sowohl an der Ankerplatte, als auch an der Druckplatte angebracht sein.

In einem gebremsten Zustand erzeugt die durch die Anpresskraft der Druckfedern erzeugte Reibung ein Bremsmoment. Das Bremsmoment verhindert beispielsweise eine unbeabsichtigte Bewegung eines Tragarmsystems, welches mittels der Lagereinheit wenigstens bezüglich eines rotatorischen Freiheitsgrad beweglich gehalten ist. Bei einer elektromagnetischen Betätigung der Lagereinheit, welche auch als Aktivierung bezeichnet werden kann, wird ein starker Elektromagnet, welcher beispielsweise fest mit dem Stator verbunden ist, mit einem Strom beaufschlagt. Das entstehende Magnetfeld wirkt der Druckkraft der Federn entgegen und zieht die Ankerplatte an. Das Anpressen des Reibbelags wird dadurch beendet und Stator und Rotor lassen sich dann mit geringem Kraftaufwand gegeneinander verdrehen. Charakteristisch bei diesem Wirkprinzip ist, dass die Anpresskraft, welche das Bremsmoment verursacht, der Kraft entspricht, welcher der Elektromagnet zum Lüften der Bremse erzeugen muss. Bei einem hohen Bremsmoment muss der Elektromagnet also entsprechend stark dimensioniert sein und die Ströme innerhalb einer Spule des Elektromagneten sind entsprechend groß. Ebenso ist der Spalt zwischen Elektromagnet und Ankerplatte für die Funktion bei hohen Bremsmomenten ausschlaggebend, da die magnetische Kraft mit dem Abstand zum Magneten stark abnimmt. Dies stellt hohe Anforderungen an die Fertigungsgenauigkeit und den Montageaufwand, insbesondere um den Spalt gering zu halten. Beispielsweise wird zur Einstellung des Spalts ein aufwendig zu fertigendes Feingewinde mit geringer Steigung zwischen Druckplatte und Gehäuse vorgesehen. Die Lage der Druckplatte am Feingewinde wird dabei nach Positionseinstellung mittels eines Klebstoffes fixiert, was eine Wartung sehr aufwendig macht.

Eine weitere bekannte konstruktive Lösung zum Festsetzen oder Bremsen einer Lagereinheit ist die Verwendung eines elastischen Druckschlauches, welcher durch eine Beaufschlagung mit einem pneumatischen Innendruck expandiert. Die Expansion führt zu einer Anpresskraft auf Rotor und Stator und erzeugt an den Wirkflächen eine Reibkraft, welche die Drehbewegung hemmt. Der Druckschlauch kann also beispielsweise die Funktion der Federn und des Elektromagneten übernehmen. Nachteilig bei dieser Lösung ist, dass bei Bewegung eines mittels der Lagereinheit gelagerten Tragarmsystems bei ungelöster Bremse ein abrasiver Verschleiß an den relativ bewegten Wirkflächen des Druckschlauchs, welcher auch als Pneumatikschlauch bezeichnet wird, auftritt. Dieser abrasive Verschleiß kann zu einer Undichtigkeit des Druckschlauchs, welcher auch als Bremsschlauch bezeichnet wird, führen. Die Lebensdauer der Lagereinheit kann somit gering sein und/oder jeweilige Wartungsintervalle können kurz sein. Ein ähnlicher Effekt kann durch Risse an jeweiligen Crimpstellen des elastischen Schlauches, an welchen dieser befestigt ist, auftreten. Die Wartung eines defekten Bremsschlauchs ist ein aufwendiger Serviceeinsatz.

Eine dritte bekannte konstruktive Lösung zum Festsetzen oder Bremsen einer Lagereinheit ist eine Friktionsbremseinheit, welche nicht lösbar ist. Hierbei wird ein dauerhaftes Bremsmoment zwischen Rotor und Stator erzeugt, welches vom Bediener nicht gelöst werden kann. Üblicherweise ist das Bremsmoment dabei so hoch gewählt, dass eine Bewegung eines mit der Lagereinheit gelagerten Tragarmsystems dennoch möglich ist, ungewollte Bewegungen aber relativ zuverlässig verhindert werden. Bei einigen Ausführungsformen ist dieses Bremsmoment nicht einstellbar, ändert sich mit fortschreitendem Verschleiß der Reibflächen und ist dann nicht mehr korrigierbar.

Den beschriebenen Lagereinheiten ist zudem gemein, dass sich diese durch eine hohe Variantenvielfalt von Bauteilen und Baugruppen auszeichnen. Auch dadurch sind diese Lagereinheiten teuer und aufwendig zu warten.

Beispielsweise sind elektromagnetisch betätigbare Lagereinheiten in der DE 10 2007 013 164 A1 und der DE 10 2012 202 366 A1 beschrieben.

Aufgabe der vorliegenden Erfindung ist es, eine Lageranordnung zu schaffen, welche die oben genannten Nachteile wenigstens teilweise überwindet.

Diese Aufgabe wird erfindungsgemäß durch eine Lageranordnung mit den Merkmalen des unabhängigen Patentanspruchs gelöst. Vorteilhafte Ausgestaltungen der Erfindung sind mit den jeweiligen Unteransprüchen angegeben.

Die Erfindung betrifft eine Lageranordnung. Die Lageranordnung kann wenigstens ein erstes Lagerelement und ein zweites Lagerelement, welche entlang einer gemeinsamen Längsachse relativ rotierbar zueinander miteinander verbunden sind, umfassen. Weiterhin kann die Lageranordnung eine Bremsvorrichtung umfassen, welche die relative Rotation der beiden Lagerelemente zueinander mittels einer durch die Bremsvorrichtung erzeugten Reibkraft hemmt, wobei die Bremsvorrichtung wenigstens ein Reibelement und wenigstens eine Spannvorrichtung aufweist, mittels welchen das Reibelement dauerhaft gegen eines der beiden Lagerelemente verspannt sein kann. Weiterhin kann die Lageranordnung eine Kupplungsvorrichtung umfassen, welche zwischen einem offenen Zustand und einem geschlossenen Zustand verstellbar ist. Die Kupplungsvorrichtung kann dabei auch als Kupplung bezeichnet werden. In dem geschlossenen Zustand kann die Bremsvorrichtung eingekuppelt sein, insbesondere in einem Kraftpfad zwischen den beiden Lagerelementen, wodurch bei einer Rotation der beiden Lagerelemente relativ zueinander diese Rotation durch die mittels der Bremsvorrichtung erzeugte Reibkraft gehemmt ist. In dem offenen Zustand kann die Bremsvorrichtung ausgekuppelt sein, wodurch die beiden Lagerelemente frei von einer Hemmung durch die Bremsvorrichtung relativ zueinander rotierbar sind.

Die Lageranordnung kann auch als Lagereinheit mit schaltbarer Friktionsbremseinheit bezeichnet werden. Die Lageranordnung kann als Schwerlastlagereinheit ausgebildet sein. Besonders geeignet kann die Lageranordnung für medizinische Geräte sein, insbesondere als Lagerung für Tragarmsysteme, wie beispielsweise Tragarme von medizinischen Gerätschaften in einem medizinischen Behandlungsraum, wie einen OP-Raum. Mittels der Lageranordnung kann wenigstens ein Tragarm beispielsweise an einer Decke des Raums und/oder einer Vorrichtung zum Halten medizinischer Gerätschaften verbunden sein. Die Lageranordnung kann also bei medizinischen Geräten verwendet werden.

Bei der Lageranordnung wird die Reibkraft zum Hemmen der Bewegung der beiden Lagerelemente zueinander bzw. die Vorspannkraft, welche die Erzeugung der Reibkraft bei der relativen Bewegung bewirkt, also dauerhaft mittels der Bremsvorrichtung bereitgestellt. Beispielsweise umfasst die Bremsvorrichtung zwei aneinander dauerhaft anliegende und gegeneinander gepresste Reibflächen, welche bei einer Bewegung der zwei Lagerelemente relativ zueinander ebenfalls relativ zueinander bewegt werden und dabei aneinander reiben. Dadurch wird die die Bewegung hemmende Reibkraft erzeugt. Aufgrund der Kupplungsvorrichtung kann jedoch ein Mitbewegen einer der beiden Reibflächen mit einem der beiden Lagerelemente ausgekuppelt werden, so dass die beiden Reibflächen nicht mehr aneinander reiben, wenn die beiden Lagerelemente relativ zueinander bewegt werden. Dadurch wird die Bremsvorrichtung aus dem Kraftfluss zwischen den zwei Lagerelementen ausgekuppelt und eine wesentlich verringerte Kraft zum relativen Bewegen der beiden Lagerelemente zueinander benötigt. In diesem Zustand muss beispielsweise nur noch die normale Reibkraft der Lageranordnung überwunden werden, welche beispielsweise durch ein Abrollen jeweiliger Kugeln eines Kugellagers, mittels welcher die Lagerelemente miteinander verbunden sind, erzeugt wird. Die Bremsvorrichtung kann also aus dem Kraftfluss zwischen den zwei Lagerelementen ausgekuppelt werden. Dies kann auch als Kopplung der Bremsvorrichtung mit den zwei Lagerelementen bezeichnet werden. Entsprechend kann die Kupplungsvorrichtung auch als Kopplungsvorrichtung bezeichnet werden.

Die Lageranordnung bietet dabei insgesamt eine Reihe von Vorteilen durch die voneinander getrennt bereitgestellte Vorspannkraft zum Erzeugen der die relative Bewegung der Lagerelemente zueinander hemmenden Reibkraft und die davon separat mögliche Einkupplung und Auskupplung, welche auch als Aktivierung und Deaktivierung der Bremsvorrichtung bezeichnet werden kann. Die Betätigungskraft, welche zum Lösen der Bremswirkung bzw. der die Rotation hemmenden Reibkraft benötigt wird, ist unabhängig von der erzeugbaren bzw. gewünschten Reibkraft. Dadurch kann beispielsweise die Kupplungsvorrichtung besonders klein, leicht und kostengünstig sein, und dennoch eine hohe Reibkraft zuverlässig bereitgestellt werden. Dabei ist weiterhin ein zuverlässiges Ein- und Auskuppeln möglich. Die erzeugbare Reibkraft ist nicht begrenzt durch die technischen Möglichkeiten zur Ein- und Auskupplung der Bremsvorrichtung. Zum Beispiel ist die erzeugbare Reibkraft nicht mehr proportional zu einer Stärke des elektromagnetischen Feldes zwischen einem Elektromagneten und einer Ankerplatte. Ebenso ist die Stärke nicht mehr proportional zu einer Permeabilität der verwendeten Werkstoffe, der Festigkeit der Verbindung zwischen einem Elektromagneten und einen Stator und/oder dem Spalt zwischen einem Elektromagneten und die durch ihn zur Ein- und Auskupplung bewegbaren Scheibe. Dadurch kann die Bremsvorrichtung konstruktiv einfach sein und dennoch hohe Bremskräfte zur Verfügung stellen. Beispielsweise kann die Bremsvorrichtung eine Reibfläche, insbesondere ohne besondere Anforderungen, aufweisen, welche mittels einer Feder dauerhaft gegen eines der Lagerelemente verspannt ist, was auch als Pressung bezeichnet werden kann.

Zudem können die Bremsvorrichtung und damit auch die Lageranordnung besonders kostengünstig sein, da nicht mehr fertigungstechnisch aufwändig ein präziser Spalt gefertigt werden muss, um bei einer Produktreihe ein einheitliches Bremsmoment sicherzustellen. Auch auf ein Feingewinde zum Einstellen eines solchen Spalts kann verzichtet werden. Zudem kann die erzeugte Bremskraft, welcher der hemmenden Reibkraft bei der Bewegung der zwei Lagerelemente relativ zueinander entspricht, bei der Endmontage der Lageranordnung oder auch beim Kunden, beispielsweise in einem Krankenhaus, einstellbar sein. Dadurch kann der Bediener immer dieselbe Bedienungskraft bei eingekuppelter Bremsvorrichtung spüren bzw. benötigen.

Außerdem können unterschiedliche Arten der Aktivierung der Bremsvorrichtung bzw. Einkupplung der Bremsvorrichtung vorgesehen werden, ohne dass die Bauweise der Bremsvorrichtung selber umfangreich modifiziert werden muss. Bei der oben beschriebenen Lageranordnung werden also die Kupplungsfunktion und die Bereitstellung der Vorspannung für die Erzeugung der hemmenden Reibkraft nicht durch ein gemeinsames Bauteil, wie beispielsweise einem Elektromagneten, bereitgestellt und/oder beeinflusst, insbesondere hinsichtlich der Auslegung der Lageranordnung. Zudem kann ein Verschleiß der Lageranordnung besonders gering sein. Wird beispielsweise ein pneumatischer Schlauch zur Verstellung der Kupplung benutzt, tritt dieser nicht notwendigerweise in Kontakt mit einem eine Reibfläche der Bremsvorrichtung aufweisenden Bauteil und/oder mit anderen relativ zueinander bewegbaren Bauteilen. Entsprechend steht der pneumatische Schlauch nicht in Kontakt mit einem relativ dazu bewegten Bauteil, welcher den Schlauch abreiben und/oder anderweitig beschädigen kann.

Die Hemmung der relativen Bewegung der beiden Lagerelemente zueinander kann einen erhöhten Kraftaufwand beim Bewegen der beiden Lagerelemente relativ zueinander im Vergleich zu einer ungehemmten Lageranordnung erfordern. Die Lageranordnung kann aber auch soweit gehemmt sein, dass diese zumindest in Bezug auf übliche Betätigungskräfte vollständig festgelegt ist. In diesem Fall kann die Reibkraft so hoch sein, dass sie durch einen Bediener nicht mehr zu überwinden ist. Vorzugsweise ist die hemmende Reibkraft so hoch, dass die beiden Lagerelemente immer noch relativ zueinander mit einem üblichen Kraftaufwand eines Benutzers bewegt werden können aber ungewollte Bewegung, beispielsweise durch ein unbeabsichtigtes Stoßen gegen einen Tragarm, unterbunden werden oder zumindest erheblich reduziert sind.

Die Reibkraft kann beispielsweise dadurch erzeugt werden, dass ein Spannelement der Spannvorrichtung der Bremsvorrichtung mit einem der beiden Lagerelemente mitbewegt wird und/oder dass ein Reibelement, welches mittels des Spannelements verspannt ist, mit dem anderen der beiden Lagerelemente mitbewegt wird. Das Spannelement kann insbesondere ein Spannelement sein, mit welchem ein als Bremsring ausgebildetes Reibelement radial außen umspannt ist und gegen eine Außenseite eines Lagerelements gepresst wird, wodurch ein Reibkraftfluss zwischen Reibelement bzw. Bremsring und Lagerelement ausgebildet ist.

Das Spannelement, das beispielsweise eine Spannschelle sein kann, kann in umlaufender Richtung in einer Vertiefung am Reibelement wenigstens teilweise aufgenommen sein. Dadurch kann das Spannelement das Reibelement auch axial sichern. Ebenso kann das Reibelement in umlaufender Richtung in einer Vertiefung an einem der Lagerelemente wenigstens teilweise aufgenommen sein. Das Reibelement und/oder das Spannelement können so axial gesichert sein. Eine Bewegung dieser Teile entlang der Längsachse kann so zuverlässig verhindert werden. Dadurch kann insbesondere auf eine axiale Sicherung in Form eines Feingewindes und/oder einer Verklebung verzichtet werden. Dadurch kann die Lageranordnung besonders kostengünstig sein und zudem besonders einfach gewartet werden. Insbesondere kann die Lageranordnung ohne Weiteres zur Wartung und/oder zum Austauschen einzelner Teile zerlegt werden.

Das Reibelement kann rotationsfest mit einem der beiden Lagerelemente in geschlossenem Zustand der Kupplungsvorrichtung verbunden sein und in einem geöffneten Zustand der Kupplungsvorrichtung von dieser rotatorisch entkoppelt sein. Jeweilige Lagerelemente können beispielsweise als Gehäuseteile der Lageranordnung ausgebildet sein, welche beispielsweise mittels eines Gleitlagers oder Kugellagers miteinander beweglich verbunden sind. Jeweilige Lagerelemente können auch Lagerteile selbst sein, wie beispielsweise jeweilige Ringe eines Kugellagers oder Gleitlagers. Eines der Lagerelemente kann beispielsweise als ein Stator ausgebildet sein. Der Stator kann beispielsweise drehfest an einem Operationstisch, einer Wand und/oder einer Decke eines OP-Saals montiert sein. Das andere der beiden Lagerelemente kann dementsprechend als ein Rotor ausgebildet sein und bezüglich dem anderen Lagerelement rotierbar an diesem befestigt sein.

Die gesamte Lageranordnung kann von einer Kunststoffverkleidung umgeben sein. Die Kunststoffverkleidung kann die Lageranordnung vor Verschmutzung schützen und/oder jeweilige Benutzer vor einem Einklemmen zwischen jeweiligen beweglichen Teilen der Lageranordnung schützen. Die Verkleidung kann beispielsweise zweiteilig ausgeführt sein, um diese einfach für eine Wartung der Lageranordnung öffnen und/oder demontieren zu können.

Die Bremsvorrichtung kann auch als Reibvorrichtung oder als Friktionsbremsvorrichtung bezeichnet werden. Die Spannkraft und damit die Reibkraft kann axial oder radial bezüglich der Längsachse der Lageranordnung wirkend sein. Ein Reibelement bzw. jeweilige Reibflächenpaare können beispielsweise am Außenumfang eines der beiden Lagerelemente, zum Beispiel an einer Außenumfangsoberfläche des Rotors, angeordnet sein. Alternativ oder zusätzlich können auch Reibflächenpaare in Längsrichtung über oder unter bzw. in Längsrichtung an einer Stirnfläche an einem der beiden Lagerelemente angeordnet sein.

Die Lageranordnung stellt eine Kupplungsvorrichtung und eine Bremsvorrichtung zur Verfügung, welche funktional getrennt voneinander ausgebildet sind und auch voneinander getrennte Bauteile aufweisen, insbesondere wenigstens voneinander getrennte bewegliche Bauteile. Die Vorspannung für die Reibkraft kann getrennt davon erzeugt werden, ob eine Kupplungsvorrichtung im geöffneten oder geschlossenen Zustand und damit die Bremsvorrichtung im Kraftpfad zwischen relativ zueinander rotierbaren Lagerelementen eingespannt ist oder nicht.

In weiterer vorteilhafter Ausgestaltung der Lageranordnung ist es vorgesehen, dass die Spannvorrichtung dazu ausgebildet ist, das Reibelement mit einer einstellbaren Vorspannkraft dauerhaft gegen eines der beiden Lagerelemente zu verspannen. Dadurch ist die Reibkraft zur Hemmung der Rotation der beiden Lagerelemente relativ zueinander einstellbar. Das Reibelement kann dabei eine Fläche eines Spannelements sein, welche beispielsweise gegen eines der beiden Lagerelemente gepresst wird, um so die Reibflächenpaare zur Verfügung zu stellen. Das Reibelement kann aber auch ein von dem Spannelement separates Teil sein. Beispielsweise kann das Reibelement ein zusätzliches Reibteil mit hoher Oberflächenrauigkeit sein, um eine hohe Reibkraft bei geringer Fläche zur Verfügung stellen zu können. Das Reibelement kann mittels der Spannvorrichtung eingespannt sein. Gleichzeitig kann das Reibelement auch als austauschbares Verschleißteil ausgebildet sein, so dass die Lageranordnung einfach und kostengünstig gewartet werden kann.

Durch die Einstellbarkeit der Reibkraft kann diese an jeweilige Benutzerwünsche und/oder einen jeweiligen Einsatz der Lageranordnung angepasst werden. Beispielsweise kann die Reibkraft an eine Länge eines Tragarms eines Tragarmsystems in einem OP-Saal angepasst werden. So kann bei einem langen Tragarm eine hohe Reibkraft eingestellt werden, sodass zum Bewegen dieses langen Tragarms im Vergleich zu einem kürzeren Tragarm aufgrund des größeren Hebelarms dennoch im Vergleich zu einer Lagerung des kürzeren Tragarms im Wesentlichen dieselbe Kraft benötigt wird. Dadurch kann die Lageranordnung eine besonders angenehm zu benutzende Halterung von medizinischen Geräten zur Verfügung stellen. Beispielsweise kann bei zwei aneinander gelagerten Tragarmen verhindert werden, dass sich der längere Tragarm an einer Decke immer zuerst bewegt. Zudem können durch die Einstellbarkeit jeweilige Fertigungsungenauigkeiten ausgeglichen werden, um so trotz jeweiliger Fertigungsabweichungen bei allen Lageranordnungen dieselbe Reibkraft zur Verfügung stellen zu können.

In weiterer vorteilhafter Ausgestaltung der Lageranordnung ist es vorgesehen, dass die Spannvorrichtung eine geteilte oder ungeteilte Spannschnelle, einen gebogenen Draht, insbesondere mit Gewinde an seinen Enden und mit einer Spannmutter verspannt, ein in einem Spannelement der Spannvorrichtung eingehaktes Stahlseil, ein Stahlseil oder Stahlband, welches mittels eines Spannelements, wie einem Kipphebel, verspannt ist, und/oder ein Endlosstahlseil oder ein Endlosstahlband, welches U-förmig um das Lagerelement gelegt ist und mit einem Spannelement an seinen Enden verspannt ist, umfasst. Das Spannelement zum Spannen des Endlosstahlseils oder Endlosstahlbands kann beispielsweise eine Verschraubung sein. Ebenso kann eine Spannschelle mittels einer Verschraubung gespannt werden. Der Draht kann beispielsweise ringförmig um eines der beiden Lagerelemente gelegt sein. Die Spannmutter kann beispielsweise einen Außensechskant aufweisen und/oder zwei Innengewinde, wobei eines der Innengewinde ein Rechtsgewinde ist und das andere ein Linksgewinde. Durch das Aufschrauben der Spannmutter auf den Draht an seinen beiden Enden kann dieser dann simpel durch eine einzige Spannmutter gegen ein Reibelement verspannt werden, wobei insbesondere beide Enden gleichzeitig gleichmäßig angezogen werden können. Den genannten Teilen ist gemein, dass diese einfach und kostengünstig sind und zudem wenigstens teilweise als günstige Standardbauteile zur Verfügung stehen. Dadurch kann die Spannvorrichtung konstruktiv einfach und kostengünstig gestaltet sein. Insbesondere kann auf aufwendige und präzise eingebaute magnetische Aktuatoren zur Aufhebung der Vorspannkraft verzichtet werden. Grundsätzlich muss die Vorspannkraft nicht komplett aufhebbar sein. Gegebenenfalls sollte die Vorspannkraft aber einstellbar sein, um so die Reibkraft einstellen zu können, wie vorher geschildert. Dies ist mit den beschriebenen Elementen besonders einfach möglich, beispielsweise indem diese fester oder weniger fest angezogen werden.

In weiterer vorteilhafter Ausgestaltung der Lageranordnung ist es vorgesehen, dass die Lageranordnung ein Gehäuse aufweist, wobei die Bremsvorrichtung und/oder die Kupplungsvorrichtung außerhalb oder innerhalb des Gehäuses angeordnet sind. Das Gehäuse kann dabei beispielsweise durch eines der oder beide der Lagerelemente gebildet sein oder diese umfassen. Innenseitig und außenseitig kann dabei beispielsweise in Bezug auf die radiale Richtung und die Längsachse definiert sein. Das Gehäuse kann beispielsweise auch Lagerflächen, in welchen die Lagerelemente aneinander abgleiten, Kugeln eines Rollenlagers und/oder jeweilige Lagerelemente wenigstens bereichsweise umgeben oder teilweise einhüllen. Dadurch ist die Lageranordnung vor Verschmutzung und ungewollten Manipulationen gut geschützt. Durch die Anordnung der Bremsvorrichtung und/oder der Kupplungsvorrichtung innerhalb des Gehäuses und/oder innerhalb der beiden Lagerelemente kann die Lageranordnung besonders kompakt sein. Durch die Anordnung außerhalb des Gehäuses bzw. der beiden Lagerelemente können die Bremsvorrichtung und/oder die Kupplungsvorrichtung besonders einfach für eine Wartung zugänglich sein. Gleichzeitig müssen so jeweilige Teile der Kupplungsvorrichtung und/oder der Bremsvorrichtung nicht an einen begrenzten Innenraum angepasst sein, wodurch einfach jeweilige Standardbauteile genutzt werden können.

In weiterer vorteilhafter Ausgestaltung der Lageranordnung ist es vorgesehen, dass die Kupplungsvorrichtung insbesondere eine radial innenseitig ausgerichtete Verzahnung von einem der beiden Lagerelemente, insbesondere dem Lagerelement, gegen welches das Reibelement nicht verspannt ist, umfasst, und eine insbesondere radiale außenseitig ausgerichtete korrespondierende Verzahnung an dem Reibelement umfasst, wobei die beiden Verzahnungen in dem geschlossenen Zustand der Kupplungsvorrichtung miteinander in Eingriff stehen und in dem geöffneten Zustand der Kupplungsvorrichtung nicht miteinander in Eingriff stehen. Wenn die Verzahnung nicht in Eingriff steht, kann dies auch als gelöster Eingriff bezeichnet werden. Mittels der Verzahnung kann ein Kraftpfad mit konstruktiv einfachen Maßnahmen zuverlässig geschlossen werden, so dass die Bremsvorrichtung in dem Kraftpfad zwischen den beiden relativ zueinander bewegten Lagerelementen eingekuppelt ist. Die Verzahnung kann dabei beispielsweise einfach in Eingriff gebracht werden, indem Zähne an einem axial in Längsrichtung beweglichen Bauteil angeordnet ist. Beispielsweise kann an dem Lagerelement, gegen welches das Reibelement nicht verspannt ist, eine axiale bewegliche Deckelscheibe angeordnet sein. Diese Deckelscheibe kann beispielsweise mit jeweiligen Führungsstiften axial geführt sein und mittels jeweiliger Federelemente, welche beispielsweise an den Führungsstiften angeordnet sind, in eine axiale Position vorgespannt sein, in welcher die Zähne der Verzahnung miteinander in Eingriff stehen. Durch ein axiales Verschieben dieser Deckelscheibe können die Zähne außer Eingriff gebracht werden, so dass die Bremsvorrichtung ausgekuppelt ist. Vorzugsweise ist das axial bewegliche Bauteil drehfest bezüglich der Längsachse mit dem Lagerelement, gegen welches das Reibelement nicht verspannt ist, verbunden. Beispielsweise kann die Deckelscheibe mittels der Führungsstifte rotatorisch an dem entsprechenden Lagerelement festgelegt sein. Die Führungsstifte können dabei auch als Führungsbolzen bezeichnet werden.

Die Verzahnung kann beispielsweise außenseitig und/oder innenseitig komplett umlaufend sein oder auch nur teilweise in Umfangsrichtung ausgebildet sein. Beispielsweise kann an dem Reibelement eine komplett außenseitig umlaufend ringförmig Verzahnung vorgesehen sein, während an dem Lagerelement, an welchem das Reibelement nicht verspannt ist, eine nur teilweise radial innenseitig umlaufende Verzahnung angeordnet ist, beispielsweise an der Deckelscheibe. Dadurch kann jederzeit ein Eingriff erreicht werden, unabhängig von der Winkelstellung der beiden Lagerelemente, während dennoch nicht zwei komplett umlaufende Verzahnungen benötigt werden. Dadurch kann die Lageranordnung besonders leicht und kostengünstig sein. Zudem können jeweilige Fertigungstoleranzen bei den Verzahnungen weniger hohe Anforderungen haben, da nicht über einen komplett umlaufenden Ring korrespondierende Passungen sichergestellt werden müssen. Die Kupplung kann durch die Verzahnung geschlossen oder freigegeben werden, womit dann auch ein Kraftfluss über die Bremsvorrichtung hergestellt werden kann oder geöffnet werden kann.

Verzahnung kann dahingehend verstanden, dass ein Teil oder Teilbereich eine außenseitige zahnförmige Oberfläche aufweist, welche mit der korrespondierenden Verzahnung bzw. zahnförmigen Oberfläche in einen kraftschlüssigen Eingriff gebracht werden kann. Die Verzahnung kann dabei aber auch nur eine solche zahnförmige Oberfläche bzw. Zähne auf einer Seite der Verzahnung bezeichnen. Der kraftschlüssige Eingriff von den beiden Verzahnungen kann auch als Verzahnungseingriff bezeichnet werden.

In weiterer vorteilhafter Ausgestaltungen der Lageranordnung ist vorgesehen, dass wenigstens eine der beiden Verzahnungen an einem in Längsrichtung beweglichen Kupplungselement angeordnet ist. Die Verzahnung kann durch Bewegen des Kupplungselements in Längsrichtung in Eingriff gebracht und aus dem Eingriff gebracht bzw. bewegt werden, wodurch die Kupplung zwischen ihrer geschlossenen und ihrer offenen Stellung verstellbar ist. Eine solche Kupplungsvorrichtung ist besonders simpel und besonders robust. Beispielsweise kann ein Kupplungsring vorgesehen sein, welcher mit dem Lagerelement, gegen das Reibelement nicht verspannt ist, rotationsfest, aber axial beweglich verbunden sein. Der Kupplungsring kann, insbesondere rotatorisch, mitgeführt sein mit diesem Lagerelement, beispielsweise mittels einer Mehrzahl von in Umfangsrichtung verteilten Führungsbolzen.

In weiterer vorteilhafter Ausgestaltung der Lageranordnung ist es vorgesehen, dass die Kupplungsvorrichtung unbetätigt in ihrem geschlossenen Zustand verbleibt, insbesondere indem die Kupplungsvorrichtung durch ein Federelement in den geschlossenen Zustand vorgespannt ist. Eine Ausgangsstellung der Kupplungsvorrichtung, in welchen diese selbsttätig zurückkehrt, kann also deren geschlossener Zustand sein. Das Federelement kann dabei beispielsweise auch Teil der Kupplungsvorrichtung sein. Beispielsweise kann der Kupplungsring mittels an jeweiligen Führungsbolzen befestigter Federelementen in einer bestimmten Axialposition vorgespannt sein, in welcher die Verzahnung in Eingriff ist. Diese axiale Position kann beispielsweise durch jeweilige Anschläge vorgegeben sein, beispielsweise jeweilige Köpfe der Führungsbolzen.

Die Kupplungsvorrichtung kann durch Betätigung, insbesondere durch Betätigung eines Aktors, aus dem geschlossenen Zustand in den offenen Zustand verstellt werden und nach Beendigung der Betätigung dann selbsttätig in den geschlossenen Zustand zurückkehren. Dadurch kann erreicht werden, dass die Lageranordnung bezüglich einer Relativbewegung der beiden Lagerelemente zueinander immer gehemmt ist, wenn die Kupplung nicht betätigt und/oder ausgefallen ist. Dadurch ist die Sicherheit erhöht, da nicht ungewollter Weise trotz nur geringen Kraftaufwands jeweilige Lagerelemente ungehemmt bewegt werden können

Vorteilhafter Weise wird dabei die Relativbewegung der beiden Lagerelemente zueinander nicht vollständig durch die Bremsvorrichtung blockiert, so dass beispielsweise auch bei einem Stromausfall und/oder eines Ausfalls des Aktors in einem OP-Saal weiterhin die Tragarme mit den daran befestigten medizinischen Gerätschaften mitbewegt werden können. Durch die Betätigung des Aktors kann dabei die Bewegungskraft, also wenn kein Ausfall vorliegt, durch Auskupplung der Bremsvorrichtung wesentlich reduziert werden, so dass die beiden Lagerelemente relativ zueinander schnell und mit geringem Kraftaufwand bewegt werden können.

Jeweilige Federelemente können beispielsweise als Druckfeder ausgebildet sein, mittels welcher das Kupplungselement in eine Position gegen einen Anschlag vorgespannt ist, der dem geschlossenen Zustand der Kupplung entspricht. Jeweilige Federelement können beispielsweise auch als Zugfeder, Spiralfeder, Tellerfeder, pneumatische Feder, hydraulische Feder oder als ein anderes Federelement ausgebildet sein. Vorzugsweise kann das Federelement potentielle Energie beim Verstellen aus dem geschlossenen Zustand in den geöffneten Zustand speichern und diese Energie zum anschließenden selbsttätigen Rückverstellen in den geschlossenen Zustand nutzen.

In weiterer vorteilhafter Ausgestaltung der Lageranordnung ist es vorgesehen, dass die Kupplung einen elektromagnetischen Aktor, wie einen Elektromagneten und/oder einen Hubmagneten, ein Antriebselement, wie einen Servomotor, einen Bowdenzug, einen hydraulischen Aktor, und/oder einen pneumatischen Aktor, wie einen mit Druck beaufschlagbaren und damit aufblasbaren Druckschlauch, zum Verstellen zwischen dem geschlossenen und dem geöffneten Zustand aufweist. Der Servomotor kann beispielsweise auch als Schrittmotor ausgebildet sein, wodurch auf einen Anschlag zum Definieren einer jeweiligen Position für den geöffneten und den geschlossenen Zustand verzichtet werden kann. Mittels der vorher genannten Aktoren kann die Kupplung einfach betätigt werden, beispielsweise in Reaktion auf ein Sensorsignal, beispielsweise einen Berührungssensor an einem Tragarm und/oder eine Betätigung einer Eingabevorrichtung, wie einen Betätigungsknopf. Bei dem Aktor kann es sich um ein übliches Standardbauteil handeln, welches auch als Katalogteil bezeichnet werden kann.

Der Aktor muss dabei nicht dazu ausgebildet sein, eine jeweilige Vorspannkraft zum Bereitstellen der Reibkraft zum Hemmen der Bewegung der beiden Lagerelemente relativ zueinander zur Verfügung zu stellen und/oder Aufheben zu können. Dadurch kann ein besonders kleiner, leichter und/oder kostengünstiger Aktor verwendet werden. Der Aktor kann von der eigentlichen Bremsfunktion der Lageranordnung unabhängig ausgewählt und ausgebildet sein. Ein Aktor kann auch als Aktuator bezeichnet werden. Ein elektromagnetischer Aktor kann beispielsweise einen Ringmagneten umfassen. Der Druckschlauch kann beispielsweise in einem entspannten Zustand einen eingekuppelten und/oder geschlossenen Zustand der Kupplung bewirken, und in einem expandierten Zustand einen ausgekuppelten bzw. geöffneten Zustand der Kupplungsvorrichtung. Dadurch kann die Kupplungsvorrichtung auch bei einer Beschädigung des Druckschlauchs selbsttätig in den geschlossenen Zustand zurückkehren.

In weiterer vorteilhafter Ausgestaltungen der Lageranordnung ist es vorgesehen, dass die Kupplungsvorrichtung eine Getriebevorrichtung umfasst, mittels welcher eine Kraft zum Verstellen zwischen dem geöffneten Zustand und dem geschlossenen Zustand übertragbar ist und/oder eine Vorspannkraft der Spannvorrichtung übertragbar ist, wobei die Getriebevorrichtung insbesondere zwei aneinander beim Verstellen an ihren jeweiligen Keilflächen abgleitende Keilelemente umfasst. Die Getriebevorichtung kann dabei als Teil der Kupplungsvorrichtung, insbesondere als Übersetzung für deren Aktuator, und/oder der Spannvorrichtung, insbesondere als Spannelement, vorgesehen sein. Entsprechend kann auch mehr als eine Getriebevorrichtung vorgesehen werden.

Mittels der Getriebevorrichtung kann ein Drehmoment eines Aktors erhöht werden, wodurch kleinere und/oder standardisierte Aktoren zum Verstellen zwischen dem geöffneten Zustand und dem geschlossenen Zustand verwendet werden können. Dies ist insbesondere bei den nur kleinen notwendigen Verstellwegen von Vorteil. Beispielsweise müssen beim Verstellen nur jeweilige Verzahnungen außer Eingriff gebracht werden, welche sich in Längsrichtung jeweils nur über wenige Millimeter erstrecken. Bei der Veränderung der Spannung, mit welcher das Reibelement gepresst wird, können mitunter noch kleinere Verstellwege, aber das Aufbringen sehr hoher Kräfte notwendig sein. Durch das Abgleiten der jeweiligen Keilelemente an ihren Keilflächen aneinander können sich diese auf einander zu bewegen oder voneinander wegbewegen. Die Keilelemente können dadurch eine Distanz ihrer Mittelpunkte zueinander verändern, wodurch eine extreme Übersetzung auf kleinem Raum ermöglicht wird. Insbesondere kann dadurch ein sehr hohes Drehmoment erreicht werden, wodurch die Kupplung auch bei hohen Wirkungen von Reibkräften und/oder hohen Verstellkräften zuverlässig in Eingriff gebracht werden kann oder dieser Eingriff wieder getrennt werden kann. Auch eine hohe Pressung des Reibelements zur Erzeugung einer hohen Reibkraft kann so einfach bereitgestellt werden. Insbesondere kann so trotz hoher notwendiger Pressungen die Spannvorrichtung noch manuell und/oder mit einfachen Werkzeugen, wie einem Schraubenzieher, eingestellt werden. Beispielsweise können die Keilelemente zwei relativ zueinander in Längsrichtung drehende Ringelemente umfassen, welche an einander zugewandten Seiten keilförmige Bereiche aufweisen, welche aneinander abgleiten. Dadurch kann die Distanz zwischen den Ringen durch Rotation veränderbar sein.

Weitere Merkmale der Erfindung ergeben sich in den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: in einer schematischen Schnittansicht eine Lageranordnung mit einer Bremsvorrichtung und einer Kupplungsvorrichtung;
- Fig. 2: in einer geschnittenen Detailansicht einen Teilbereich der Lageranordnung gemäß Fig. 1;
- Fig. 3: in einer schematischen Perspektivansicht die Lageranordnung gemäß Fig. 1;
- Fig. 4: in einer schematischen Schnittansicht einen Teilbereich der Lageranordnung gemäß Fig. 3; und
- Fig. 5: in einer schematischen Perspektivansicht ein weiteres Ausführungsbeispiel der Lageranordnung.

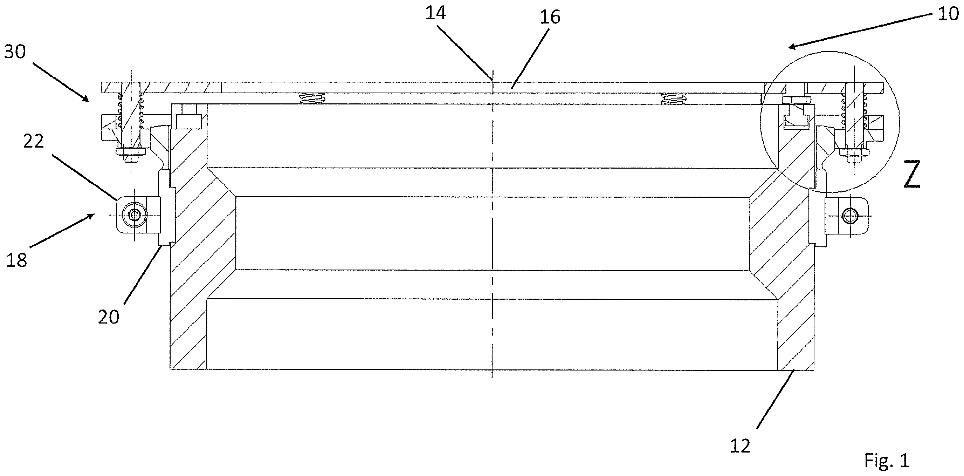

Fig. 1 zeigt in einer schematischen Schnittansicht eine Lageranordnung 10, welche ein erstes Lagerelement 12 umfasst, welches vorliegend als Rotor ausgebildet ist. Weiterhin umfasst die Lageranordnung 10 ein relativ dazu um die Längsachse 14 drehbares zweites Lagerelement, welches vorliegend als Stator ausgebildet ist und in Fig. 1 nicht dargestellt ist. Zudem umfasst die Lageranordnung 10 eine Deckelscheibe 16, welche rotationsfest mit dem Stator verbunden ist.

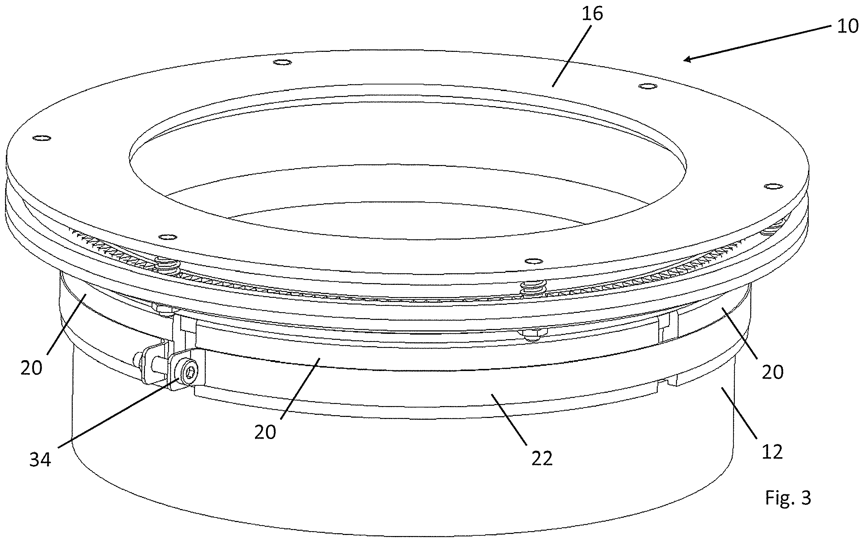

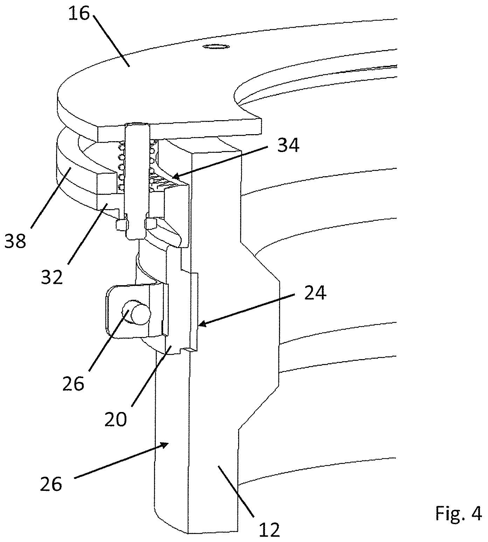

Weiterhin weist die Lageranordnung 10 eine Bremsvorrichtung 18 auf, welche einen Bremsring 20 und eine Spannschnelle 22 umfasst. Wie insbesondere gut in den schematischen Perspektivansichten der Fig. 3 und Fig. 4 zu erkennen ist, wobei die Lageranordnung 10 in Fig. 4 nur teilweise dargestellt und geschnitten gezeigt ist, ist der Bremsring 20 dabei mittels der Spannschnelle 22 in einer radial umlaufenden Vertiefung 24 einer Außenumfangsfläche 26 des Lagerelements 12 wenigstens teilweise aufgenommen. Dadurch ist der Bremsring 20 axial gesichert und kann nicht entlang der Längsachse 14 bewegt werden. Wie zudem in Fig. 3 weiterhin zu erkennen ist, besteht der Bremsring 20 aus mehreren Teilstücken, wodurch er leicht montierbar und demontierbar ist. Der Bremsring 20 kann dabei ein Verschleißteil sein oder Verschleißteile aufweisen, welche beim Erzeugen einer Reibkraft abgerieben werden. Die Spannschnelle 22 verspannt dabei den Bremsring 20 dauerhaft gegen das Lagerelement 12 und kann auch als Teil einer Spannvorrichtung bezeichnet werden. Zum Zwecke der Verspannung ist die Spannschnelle 22 mittels einer Verschraubung 28 zusammenziehbar, um so die Vorspannung zu erhöhen oder zu verringern und damit den Bremsring 20 stärker oder weniger stark an das Lagerelement 12 zu pressen. Dadurch kann eine jeweilige Reibkraft eingestellt werden, welche entsteht, wenn der Rotor und der Stator der Lageranordnung 10 relativ zueinander um die Längsachse 14 gedreht werden. Diese Reibkraft wirkt dabei dieser Rotation entgegen, was auch als Bremsung oder Hemmung der Rotation bezeichnet werden kann.

Die Bremsvorrichtung 18 bzw. der Bremsring 20 können dabei in Verbindung mit der Deckelscheibe 16 stehen, wodurch der Bremsring 20 relativ zu dem Lagerelement 12 um die Längsachse 14 bewegt wird, wenn der Rotor bzw. das Lagerelement 12 bezüglich des Stators rotiert. Der Bremsring 20 kann beispielsweise ohne Rotation in derselben Position wie das nicht gezeigte zweite Lagerelement verbleiben, wenn das Lagerelement 12 um die Längsachse 14 rotiert. Dadurch wird aufgrund des gegen die Außenumfangsfläche 26 des Lagerelements 12 verspannten Bremsrings 20 zwischen jeweiligen dort aneinander gepressten Flächen eine Bremskraft erzeugt, welche die Rotation hemmt. Dadurch ist ein erhöhter Kraftaufwand zum Bewegen des Rotors notwendig. Dies ist beispielsweise sinnvoll, falls mit dem Rotor ein Tragarm einer Haltevorrichtung für medizinische Geräte verbunden ist. Dadurch können die medizinischen Geräte präzise und zuverlässig bewegt werden, zudem kann die Lageranordnung 10 so ohne eine auf die medizinischen Geräte ausgeübte Kraft und/oder entgegen der Schwerkraft in einer jeweiligen Stellung verbleiben. Gleichzeitig kann die Reibkraft so eingestellt werden, dass ungewollte Bewegung, beispielsweise durch ein versehentliches Stoßen gegen den Tragarm, wesentlich reduziert werden oder sogar gänzlich verhindert werden.

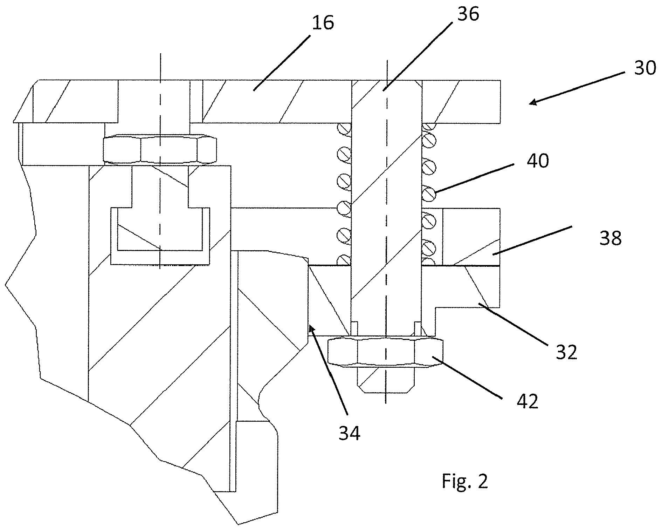

Gegebenenfalls ist jedoch eine schnelle Bewegung mit geringem Kraftaufwand gewünscht. In diesem Fall kann eine Kupplungsvorrichtung 30, welche insbesondere im Detail in Fig. 2 gezeigt ist, welche den mit Z markierten Ausschnitt der Fig. 1 vergrößert darstellt, zwischen einem offenen Zustand und einen geschlossenen Zustand verstellt werden. Die Kupplungsvorrichtung ermöglicht damit ein Ein- und Ausschalten der Bremsfunktion. In den Figuren ist dabei jeweils der geschlossene Zustand gezeigt. In dem geschlossenen Zustand ist die Bremsvorrichtung 18 eingekuppelt, wodurch bei einer Rotation des Rotors bezüglich des Stators die Bewegung des Rotors durch die mittels der Bremsvorrichtung 18 erzeugte Reibkraft gehemmt ist. Die durch die Bremsvorrichtung 18 erzeugte Reibkraft wirkt also der Rotation entgegen. Ein Kraftfluss zwischen der Deckelscheibe 16 und dem Lagerelement 12 ist dabei durch die Kupplungsvorrichtung 30 geschlossen. Zu diesem Zweck umfasst die Kupplungsvorrichtung 30 einen Kupplungsring 32, welcher radial innenseitig eine Verzahnung an dem Bereich 34 aufweist, welche dort in Eingriff mit einer radial außenseitigen Verzahnung des Bremsrings 20 steht. Der Eingriff kann dabei nur in einem Umfangsbereich vorgesehen sein oder aber auch durch jeweils komplett umlaufende Verzahnungen gebildet sein.

Soll die Kupplungsvorrichtung 30 in den offenen Zustand verstellt werden, muss der Eingriff der jeweiligen Verzahnungen gelöst werden. Im vorliegenden Beispiel muss dafür der Kupplungsring 32 in Längsrichtung nach oben entlang der Längsachse 14 bewegt werden. Zu diesem Zweck ist der Kupplungsring 32 rotationsfest aber axial beweglich mit jeweiligen Führungsbolzen 36 an der Deckelscheibe 16 befestigt. Die Führungsbolzen 36 können aber auch unmittelbar am Stator befestigt sein, wodurch auf eine Deckelscheibe verzichtet werden kann. Weiterhin umfasst zu diesem Zweck die Kupplungsvorrichtung 30 einen Elektromagneten 38. Der Elektromagnet 38 ist mit dem Kupplungsring 32 verbunden. Bei Aktivierung des Elektromagneten 38, beispielsweise durch Anlegen einer elektrischen Spannung wird der Elektromagnet 38 durch die so erzeugte Magnetkraft, zusammen mit dem Kupplungsring 32, an die Deckelscheibe 16 gezogen. Dadurch wird der Kupplungsring 32 entlang der Längsachse 14 nach oben bewegt, wodurch der Eingriff der Verzahnung in dem Bereich 34 gelöst wird. In dem nunmehr hergestellten offenen Zustand ist die Bremsvorrichtung 18 ausgekuppelt, wodurch der Rotor frei von einer Hemmung durch die Bremsvorrichtung 18 relativ zu dem Stator um die Längsachse 14 rotiert werden kann. Diese Rotation ist dann nur noch insofern gehemmt, als jeweilige andere Reibkräfte in der Lageranordnung 10 vorhanden sein können. Beispielsweise kann eine gewisse Hemmung durch ein Abrollen und/oder Abgleiten jeweiliger Kugeln eines Kugellagers oder jeweiliger Gleitflächen der Lageranordnung 10 entstehen. Im offenen Zustand kann der Rotor relativ zu dem Stator schnell und mit geringem Kraftaufwand bewegt werden.

Aus Sicherheitsgründen, insbesondere bei einem Einsatz der Lageranordnung 10 in einem OP-Saal zum Tragen jeweiliger medizinischer Geräte mittels der Lageranordnung gelagerter Tragarme, ist die Kupplungsvorrichtung 30 in ihren geschlossenen Zustand vorgespannt. Die Lageranordnung 10 bzw. die Kupplungsvorrichtung kehrt also selbsttätig in ihren geschlossenen Zustand zurück, sobald der Elektromagnet 38 deaktiviert wird. Dafür umfasst die Kupplungsvorrichtung 30 jeweilige Druckfedern 40, welche auf bzw. an den jeweiligen Führungsbolzen 36 angeordnet sind. Durch diese Positionierung der Druckfedern 40 werden diese durch die Führungsbolzen 36 mitgehalten und sind zudem besonders bauraumsparend untergebracht. Mittels der Druckfedern 40 wird der Kupplungsring 32 gegen jeweilige auf den Führungsbolzen 36 aufgesetzte Muttern 42 gedrückt. Die jeweiligen Muttern 42 dienen hier als Anschlag und geben eine Position für den geschlossenen Zustand vor, in welchem die jeweiligen Verzahnungen des Kupplungsrings 32 und des Bremsrings 20 in dem Bereich 34 in Eingriff miteinander stehen. Vorzugsweise sind die jeweiligen Druckfedern 40 dabei so dimensioniert, dass die Kupplungsvorrichtung 30 lageunabhängig, also auch entgegen der Schwerkraft, in dem geschlossenen Zustand verbleibt.

Bei der Lageranordnung 10 ist die Betätigungskraft, welche zum Lösen der Bremswirkung benötigt wird, unabhängig von der der eingestellten Vorspannung zum Erzeugen der Reibkraft. Zum Auskuppeln muss also nicht die Vorspannung, welche auf den als Reibelement ausgebildeten Bremsring 20 mit der Spannschnelle 22 wirkt, überwunden werden. Auch muss diese Vorspannung nicht aufgehoben oder gelöst werden. Stattdessen muss beispielsweise nur die Federkraft der jeweiligen Druckfedern 40 und eine Reibung der Verzahnung in dem Bereich 34 überwunden werden. Die Kräfte, welche zum Auskuppeln der Bremsvorrichtung 18 notwendig sind, können so wesentlich kleiner sein. Entsprechend kann beispielsweise auch ein sehr kleiner und preiswerter Elektromagnet 38 verwendet werden.

Gleichzeitig können jeweilige effektiv wirkende Größen jeweiliger Bremsflächenpaare, also die verspannten Kontaktflächen zwischen Bremsring 20 und Lagerelement 12, unabhängig von der Kupplungsvorrichtung 30 gestaltet werden. Dadurch kann bei gleicher Anpresskraft ein hohes Bremsmoment erreicht werden bzw. bei geringer Anpresskraft ein gleiches Bremsmoment wie bei einer Lageranordnung, bei welcher nur kleine Flächen im Bereich einer Kupplung zur Erzeugung der Reibkraft zur Verfügung stehen. Bei einer großen Fläche und dazu korrespondierend nur geringen Pressung zum Erzeugen eines ansonsten gleichen Bremsmoments kann der abrasive Verschleiß der Bremsvorrichtung 18 besonders gering sein. Da die Kupplungsvorrichtung 30 selber nicht zum Erzeugen der hemmenden Reibkraft benötigt wird, kann diese aus kostengünstigen und leichten Bauteilen bestehen, beispielsweise aus Kunststoffbauteilen. Die Kupplungsvorrichtung unterliegt nur dem geringen Verschleiß beim Ein- und Auskuppeln und wird nicht bei der eigentlichen Erzeugung der Reibkraft weiter verschlissen. Deswegen kann die Verzahnung beispielsweise als Kunststoffverzahnung ausgebildet werden, während dennoch eine hohe Reibkraft aufgrund der Vorspannung mit einer Metallspannschnelle 22 erreicht werden kann.

Die erzeugbare Bremskraft ist nicht begrenzt durch die technischen Möglichkeiten, die Kupplungsvorrichtung 30 zu öffnen bzw. auszukuppeln. Insbesondere muss kein präziser Spalt zwischen dem Elektromagneten 38 und der Deckelscheibe 16 vorgesehen sein, damit ein vorgegebenes Bremsmoment eingehalten werden kann, da der Elektromagnet 38 selber nicht die hemmenden Reibkraft vorgibt. Die erzeugte Bremskraft ist einfach durch Drehen an der Verschraubung 28 durch einen Benutzer einstellbar, beispielsweise mittels eines Schraubenziehers. Grundsätzlich kann zudem eine Vielzahl von Aktuatoren mit unterschiedlichen Wirkprinzipien anstelle des Elektromagneten 38 zum Verstellen der Kupplungsvorrichtung 30 benutzt werden.

Wie zudem in den Figuren zu erkennen ist, ist sowohl die Kupplungsvorrichtung 30 als auch die Bremsvorrichtung 18 radial außenseitig zu dem Lagerelement 12 angeordnet. Dadurch ist beispielsweise die Schraubverbindung 28 besonders gut für das Verstellen der Vorspannung und damit ein einfaches Einstellen der hemmenden Reibkraft zugänglich. Ebenso ist der Bremsring 20 leicht zugänglich, so dass dieser bei Verschleiß einfach ausgetauscht werden kann. Durch die Schraubverbindung 28 kann die Vorspannkraft nach einem Abrieb des Bremsrings 20 zudem einfach nachjustiert werden, so dass die hemmende Bremskraft durch Abrasion nicht über die Zeit zwangsläufig vermindert wird. Auch die Kupplungsvorrichtung 30 ist so einfach für eine Wartung zugänglich. Zudem können jeweilige Teile der Bremsvorrichtung 18 und der Kupplungsvorrichtung 30 standardisiert sein, da außenseitig der Einbauraum kaum begrenzt ist. Jeweilige Bauteile außenseitige Bauteile müssen nur geringfügig auf einen zur Verfügung stehenden Bauraum abgestimmt werden.

Alternativ kann aber auch die Kupplungsvorrichtung 30 und/oder die Bremsvorrichtung 18 radial in Bezug auf die Längsachse 14 innenseitig zu dem Lagerelement 12 und/oder dem Stator und/oder der Deckelscheibe 16 angeordnet sein. Dadurch kann die Kupplungsvorrichtung 30 und/oder die Bremsvorrichtung 18 vor ungewollten Manipulationen, Beschädigungen und/oder Verschmutzung gut geschützt sein. Zudem kann so beispielsweise verhindert werden, dass sich ein Benutzer an dem Spalt der Kupplungsvorrichtung 30 einen Finger einklemmt, ohne dass eine zusätzliche Hülle bzw. ein Gehäuse notwendig ist. Zudem kann die Lageranordnung 10 so besonders klein sein. Das Lagerelement 12 kann dabei auch als Teil eines Lagergehäuses ausgebildet sein, ebenso wie der Stator und/oder die Deckelscheibe 16.

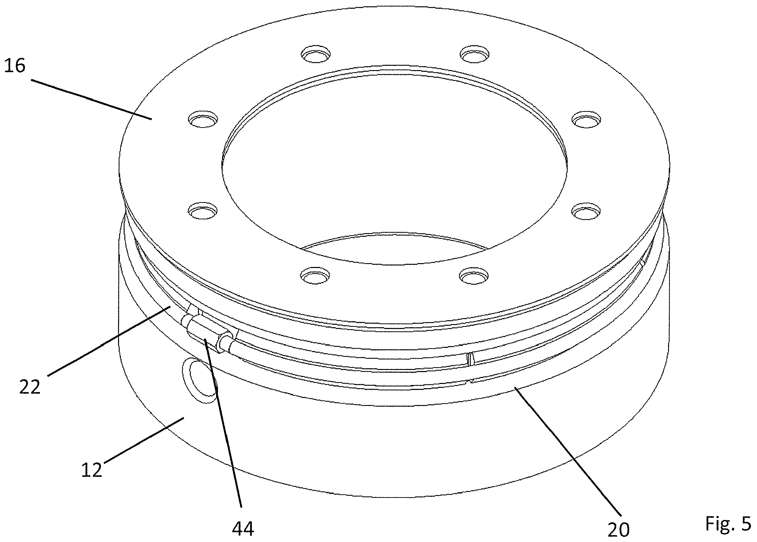

Fig. 5 zeigt in einer schematischen Perspektivansicht ein weiteres Ausführungsbeispiel der Lageranordnung 10. Die Spannvorrichtung 22 ist hier anders gestaltet. Statt eines Stahlbands mit der Verschraubung 28 wird hier zum Spannen des Bremsrings 20 ein Draht verwendet, welcher an seinen beiden Enden gegenläufige Gewinde aufweist, und eine Gewindemutter 44. Die Gewindemutter 44 weist dabei entsprechende gegenläufige Innengewinde auf, sodass der Draht gleichmäßig an beiden Enden zusammengezogen oder gelockert werden kann. Außenseitig ist die Gewindemutter 44 als Sechskant ausgebildet, sodass diese einfach mit einem entsprechenden Schlüssel zur Verstellung der Vorspannkraft gedreht werden kann. Der Bremsring 20 weist in dieser Ausführungsform eine umlaufende Vertiefung auf, um den Draht axial zu sichern. Der Draht kann beispielsweise eine Dicke von 5 mm bis 9 mm aufweisen.

### Bezugszeichenliste

- 10: Lageranordnung
- 12: Lagerelement
- 14: Längsachse
- 16: Deckelscheibe
- 18: Bremsvorrichtung
- 20: Bremsring
- 22: Spannschnelle
- 24: Vertiefung
- 26: Außenumfangsfläche
- 28: Verschraubung
- 30: Kupplungsvorrichtung
- 32: Kupplungsring
- 34: Bereich
- 36: Führungsbolzen
- 38: Elektromagnet
- 40: Druckfeder
- 42: Mutter
- 44: Gewindemutter

## Patentansprüche

1. Lageranordnung (10) mit wenigstens einem ersten Lagerelement (12) und einem zweiten Lagerelement, welche entlang einer gemeinsamen Längsachse (14) relativ rotierbar zueinander miteinander verbunden sind, wobei die Lageranordnung (10)
eine Bremsvorrichtung (18) umfasst, welche die relative Rotation der beiden Lagerelemente (12) zueinander mittels einer durch die Bremsvorrichtung (18) erzeugten Reibkraft hemmt, wobei die Bremsvorrichtung (18) wenigstens ein Reibelement (22) und wenigstens eine Spannvorrichtung (22) aufweist, mittels welcher das Reibelement (20) dauerhaft gegen eines der beiden Lagerelemente (12) verspannt ist,
und einer Kupplungsvorrichtung (30), welche zwischen einem offenen Zustand und einem geschlossenen Zustand verstellbar ist,
wobei in dem geschlossenen Zustand die Bremsvorrichtung (18) eingekuppelt ist, wodurch bei einer Rotation der beiden Lagerelemente (12) relativ zueinander diese Rotation durch die mittels der Bremsvorrichtung (18) erzeugten Reibkraft gehemmt ist, und
wobei in dem offenen Zustand die Bremsvorrichtung (18) ausgekuppelt ist, wodurch die beiden Lagerelemente (12) frei von einer Hemmung durch die Bremsvorrichtung (18) relativ zueinander rotierbar sind.

2. Lageranordnung (10) nach Anspruch 1, wobei
die Spannvorrichtung (22) dazu ausgebildet ist, das Reibelement (20) mit einer einstellbaren Vorspannkraft dauerhaft gegen eines der beiden Lagerelemente (12) zu verspannen.

3. Lageranordnung (10) nach einem der Ansprüche 1 oder 2, wobei
die Spannvorrichtung (22) eine geteilte oder ungeteilte Spannschelle (22), einen gebogenen Draht, insbesondere mit Gewinde an seinen Enden und mit einer Spannmutter verspannt, ein in einem Spannelement der Spannvorrichtung (22) eingehaktes Stahlseil, ein Stahlseil oder Stahlband, welches mittels eines Spannelements wie einem Kipphebel verspannt ist und/oder einem Endlosstahlseil oder Endlosstahlband, welches ringförmig um das Lagerelement gelegt ist und mit einem Spannelement an seinen Enden verspannt ist, umfasst.

4. Lageranordnung (10) nach einem der vorhergehenden Ansprüche, wobei
die Lageranordnung (10) ein Gehäuse aufweist, wobei die Bremsvorrichtung (18) und/oder die Kupplungsvorrichtung (30) außerhalb oder innerhalb des Gehäuses angeordnet sind.

5. Lageranordnung (10) nach einem der vorhergehenden Ansprüche, wobei
die Kupplungsvorrichtung (30) eine insbesondere radial innenseitig ausgerichtete Verzahnung an einem der beiden Lagerelemente (12), insbesondere an dem Lagerelement, gegen welches das Reibelement nicht verspannt ist, umfasst, und eine insbesondere radial außenseitig ausgerichtete korrespondierende Verzahnung an dem Reibelement (20) umfasst, wobei die beiden Verzahnungen in dem geschlossenen Zustand der Kupplungsvorrichtung (30) miteinander in Eingriff stehen und in dem geöffneten Zustand der Kupplungsvorrichtung (30) nicht miteinander in Eingriff stehen.

6. Lageranordnung (10) nach Anspruch 5, wobei
wenigstens eine der beiden Verzahnungen an einem in Längsrichtung beweglichen Kupplungselement (32) angeordnet ist.

7. Lageranordnung (10) nach einem der vorhergehenden Ansprüche, wobei
die Kupplungsvorrichtung (30) unbetätigt in ihrem geschlossenen Zustand verbleibt, insbesondere indem die Kupplungsvorrichtung (30) durch ein Federelement (40) in den geschlossenen Zustand vorgespannt ist.

8. Lageranordnung (10) nach einem der vorhergehenden Ansprüche, wobei
die Kupplungsvorrichtung (30) einen elektromagnetischen Aktor, wie einen Elektromagneten (38) oder einen Hubmagneten, ein Antriebselement wie einen Servomotor, einen Bowdenzug, einen hydraulischen Aktor, und/oder einen pneumatischen Aktor, wie einen mit Druck beaufschlagbaren und damit aufblasbaren Druckschlauch, zum Verstellen zwischen dem geschlossenen und geöffneten Zustand aufweist.

9. Lageranordnung (10) nach einem der vorhergehenden Ansprüche, wobei
die Kupplungsvorrichtung (30) eine Getriebevorrichtung umfasst, mittels welcher eine Kraft zum Verstellen zwischen dem geöffneten Zustand und dem geschlossenen Zustand übertragbar ist und/oder eine Vorspannkraft der Spannvorrichtung (22) übertragbar ist, wobei die Getriebevorrichtung insbesondere zwei aneinander beim Verstellen an ihren jeweiligen Keilflächen abgleitende Keilelement umfasst.
